Europäisches Patentamt

**European Patent Office**

**Office européen des brevets**

(19)

(11) Veröffentlichungsnummer: **0 059 784**
**B1**

(12) **EUROPÄISCHE PATENTSCHRIFT**

(45) Veröffentlichungstag der Patentschrift:
21.05.86

(51) Int. Cl.⁴: **A 61 M 5/32**, A 61 M 5/14

(21) Anmeldenummer: 81108250.2

(22) Anmeldetag: 13.10.81

(54) Injektionsvorrichtung zur Blockade peripherer Nerven, z.B. zur Plexusanästhesie.

(30) Priorität: 07.03.81 DE 8106577 U

(43) Veröffentlichungstag der Anmeldung:
15.09.82 Patentblatt 82/37

(45) Bekanntmachung des Hinweises auf die Patenterteilung:
21.05.86 Patentblatt 86/21

(84) Benannte Vertragsstaaten:
AT BE CH DE FR GB IT LI LU NL SE

(56) Entgegenhaltungen:
FR - A - 2 067 542
FR - A - 2 354 104
GB - A - 205 105
GB - A - 1 311 468

(73) Patentinhaber: Zenz, Michael, Dr., Am
Meersmannufer 28, D-3000 Hannover 58 (DE)

(72) Erfinder: Zenz, Michael, Dr., Am Meersmannufer 28,
D-3000 Hannover 58 (DE)

(74) Vertreter: Zenz, Joachim Klaus et al, Am Ruhrstein 1,
D-4300 Essen 1 (DE)

LIBER, STOCKHOLM 1986

**Beschreibung**

Die Erfindung betrifft eine Injektionsvorrichtung zur Blockade peripherer Nerven, z. B. zur plexusanästhesie, mit einer Injektionsnadel aus Metall, die an einem Ende mit einer Nadelspitze und am entgegengesetzten Ende mit einen Sockelteil zum Anschluß einer Spritze versehen ist.

Plexusblockaden werden in der Anästhesie beispielsweise bei Operationen an Arm und Hand vor allem dann angewandt, wenn dringliche Operationen bei nicht nüchternen patienten durchgeführt werden müssen. Die unbestrittenen Vorteile der plexusblockade wurden jedoch dadurch relativiert, daß es unter Umständen zu schwerwiegenden Verletzungen und Beschädigungen der Nervenfasern und der Blutgefäße, sowie zu degenerativen Veränderungen und Diskontinuitäten der Fasern durch die Spitze der Injektionsnadel bzw. -kanüle kommen kann. Plexusblockaden mit sogenannten immobilen Nadeln waren daher nur in Ausnahmefällen zu rechtfertigen und setzten eine relativ große Erfahrung des Anästhesisten voraus.

Der Erfindung liegt die Aufgabe zugrunde, eine Injektionsvorrichtung der eingangs genannten Art zur Verfügung zu stellen, bei der die Gefahr einer Beschädigung der Nervenfasern oder Verletzung der Blutgefäße durch die Injektionsnadelspitze weitgehend beseitigt ist und eine zusätzliche Hilfe durch deutliche Identifizierung des plexus geboten wird.

Bei der Lösung dieser Aufgabe geht die Erfindung von der Erkenntnis aus, daß für die Gefahren von Beschädigungen und Verletzungen der Nervenfasern und Blutgefäße die scharfe Spitze herkömmlicher Injektionsnadeln bzw. -kanülen ursächlich ist, die eine Anschliffffläche besitzen, die um weniger als 20° zur Kanülenachse geneigt ist. Von derart ausgeprägten Spitzen werden selbst dünne Nervenfasern und Blutgefäße "aufgespießt", ohne ihnen ausweichen zu können.

Die Lösung der o. g. Aufgabe besteht erfindungsgemäß darin, daß die Nadelspitze durch eine unter einem Winkel von 40 bis 60° zur Nadel- bzw. Kanülenachse geneigte Anschliffffläche gebildet ist. Aufgrund des relativ stumpfen Winkels der Anschliffffläche zur Kanülenachse bzw. zur Kanülenmantelfläche rollt ein Nerv vor der sich nähernden Injektionsnadel bzw. -kanüle bzw. vor deren Anschliffffläche weg, so daß die Nadelspitze ohne Beschädigungen oder Verletzungen der Nerven an diesen vorbeibewegt wird. Ein weiterer wesentlicher Vorteil des relativ stumpfen Anschliffs der Injektionsnadelspitze besteht darin, daß die Gefäßnervenscheide, die bei der plexusblockade von der Injektionsnadel durchstoßen werden muß, nur mit einem spürbaren Widerstand punktiert werden kann. Beim Durchstoßen der Gefäßnervenscheide entsteht aufgrund dieses Widerstands ein spürbarer "Klick", der ein zuverlässiges Zeichen dafür ist, daß die Kanülenspitze in die Nervenscheide eingedrungen ist und ihre Injektionssollage erreicht hat.

Da die Injektionskanüle eine Größe von üblicherweise 25 x 0,55 mm (24 Gauge) hat, können die Gewebe in der Regel auch bei der erfindungsgemäß vorgesehenen relativ stumpfen Anschliffffläche problemlos durchstoßen werden. Gegebenenfalls wird zum schmerzfreien Einführen der Kanüle eine Hautquaddel an der Hautpunktionsstelle verwendet.

Zur Benutzung der erfindungsgemäßen Injektionskanüle als "immobile Nadel" ist ein etwa 25 bis 35 cm langer Kunststoffschlauch mit Hilfe eines Luer-lok-Anschlusses mit dem Sockelteil der Injektionsnadel formschlüssig und flüssigkeitsdicht verbunden. An dem entgegengesetzten Ende des Schlauchs wird die Injektionsspritze ebenfalls über einen Luer-lok-Anschluß befestigt. Mit Hilfe einer solchen relativ kurzen und flexiblen Verlängerung kann die Injektionskanüle unbeeinflußt vom palpieren in Stellung gehalten werden.

Im folgenden wird die Erfindung anhand eines in der Zeichnung dargestellten Ausführungsbeispiels näher erläutert. In der Zeichnung zeigen:

Fig. 1 eine schematische Darstellung eines Ausführungsbeispiels der neuen Injektionsvorrichtung ohne Injektionsspritze; und

Fig. 2 eine stark vergrößerte Ansicht auf den die Nadelspitze bildenden Abschnitt der Injektionsnadel, gesehen in der Ebene der Anschliffffläche.

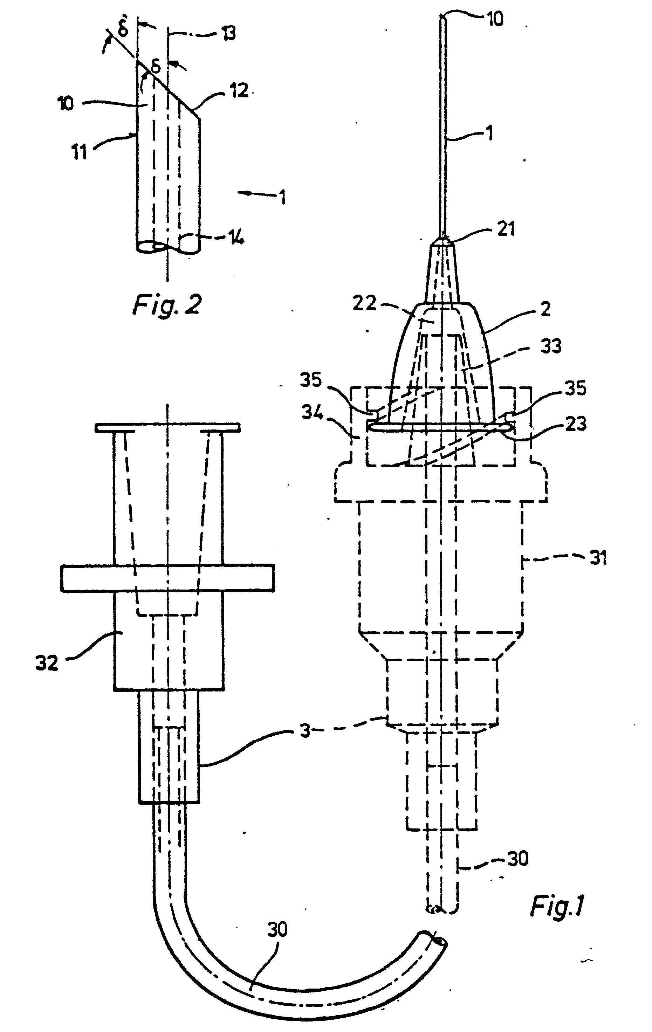

Die in Figur 1 schematisch dargestellte Injektionsvorrichtung weist eine im folgenden mit Kanüle bezilchnete Injektionsnadel 1 aus Metall auf, die an einem Ende mit einem Sockelteil 2 aus Kunststoff versehen ist. Mit dem Sockelteil 2 kann eine in der Zeichnung nicht dargestellte Injektionsspritze entweder direkt über einen Luer-lok-Anschluß oder über eine als Ganze mit 3 bezeichnete Verlängerung angeschlossen werden.

Die Kanüle 1 hat am freien Ende eine Nadelspitze 10, die durch eine die Umfangsfläche 11 schneidende Anschliffffläche 12 gebildet ist (Figur 2). Die Schnittstelle zwischen der Anschliffffläche 12 und der Mantelfläche 11 der Kanüle 1 kann vor allem am äußersten Ende leicht abgerundet sein. Die Anschliffffläche 12 verläuft unter einem relativ stumpfen Winkel $\delta$ zur Kanülenachse 13, so daß auch der die Nadelspitze bildende Scheitelwinkel $\delta' = \delta$ relativ stumpf ist. Bei einem in der praxis erprobten Ausführungsbeispiel ist $\delta$ etwa 45°; der Winkel $\delta$ liegt im Bereich zwischen 40 und 60°. Die Größe der Nadel 1 ist bei einem in der praxis erprobten Beispiel 25 x 0,55 mm (24 Gauge), so daß bei einem Anschliffwinkel $\delta$ bzw. $\delta' = 45°$ die Anschliffffläche 12 0,55 mm der Kanülenlänge überspannt.

Der Sockelteil 2 ist mit dem der Nadelspitze 10 entgegengesetzten Kanülenende an der mit 21 bezeichneten Stelle verkittet. Die Kanülenbohrung 14 (Figur 2) mündet in einen Aufnahmekonus 22 des Sockelteils 2. Der Aufnahmekonus 22 ist von einem Befestigungsflansch 23 begrenzt, der an einem Luer-lok-Anschluß bei der formschlüssigen Kupplung entweder der Injektionsspritze oder der Schlauchverlängerung 3 beteiligt ist.

Mit Hilfe der in Figur 1 dargestellten Schlauchverbindung 3 kann die Kanüle 1 als immobile Nadel verwendet werden und wird dadurch für die Blockade peripherer Nerven besonders geeignet.

Die Verlängerung 3 besteht aus einem Kunststoffschlauch 30 bei einer Länge von etwa 20 bis 35 cm, dessen Enden jeweils mit komplementären Anschlußsüücken 31, 32 einer Luer-lok-Kupplung verbunden sind. Das Anschlußstück 31 wird mit dem Sockelteil 2 gekuppelt. Dabei greift ein Abgabestutzen 33 passend in den Aufnahmekonus 22 ein, wobei eine dichte Verbindung zwischen den ineinandergreifenden Teilen hergestellt wird. Der dazu erforderliche Axialdruck wird von einer zum Kupplungsstuck 31 gehörigen Schraubkappe 34 aufgebracht, die mit schraubenlinienförmig verlaufenden Innenrippen 35 den Befestigungsflansch 23 des Sockelteils 2 hintergreifen.

Das Kupplungsstück 32 am entgegengesetzten Ende des Schlauchs 30 hat eine ähnliche Gestaltung wie der Sockelteil 2 der Kanüle, so daß mit dem Kupplungsteil 32 der Abgabeabschnitt der Injektionsspritze ebenso gekuppelt werden kann wie mit dem Sockelteil 2. Aufgrund der Verbindung der Injektionsnadel 1 über die Verlängerung 3 mit einer in der Zeichnung nicht dargestellten Injektionsspritze kann die Kanüle 1 auch beim palpieren an der Sollstelle ruhig gehalten werden und wird von Bewegungen der Spritze nicht unmittelbar beeinträchtigt. Wesentlich ist, daß die Länge des Schlauchs 30 mit den Kupplungsstücken 31 und 32 gerade so groß gewählt wird, nämlich nicht länger als etwa 35 cm, daß die Verlängerung als solche bei der Benutzung der Injektionsvorrichtung nicht stört, andererseits aber Beeinträchtigungen der Lage der Kanüle 1 durch unvermeidbare Bewegungen der Spritze nicht auftreten können.

**Patentansprüche**

1. Injektionsvorrichtung zur Blockade peripherer Nerven, z. B. zur Plexusanästhesie, mit einer Injektionsnadel (1) aus Metall, die an einem Ende mit einer Nadelspitze (10) und am entgegengesetzten Ende mit einem Sockelteil (2) zum Anschluß einer Spritze versehen ist, dadurch gekennzeichnet, daß die Nadelspitze (10) durch eine unter einem Winkel (δ) von 40 bis 60° zur Kanülenachse (13) geneigte Anschliffffläche (12) gebildet ist.

2. Injektionsvorrichtung nach Anspruch 1, dadurch gekennzeichnet, daß der Winkel (δ) der Nadelspitze (10) im Bereich zwischen 45 und 50° liegt.

3. Injektionsvorrichtung nach Anspruch 1 oder 2, dadurch gekennzeichnet, daß das äußerste Ende der Nadelspitze (10) leicht abgerundet bzw. abgestumpft ist.

4. Injektionsvorrichtung nach einem der Ansprüche 1 bis 3, dadurch gekennzeichnet, daß ein Kunststoffsockelteil (2) mit dem der Nadelspitze (10) entgegengesetzten Nadelende verklebt oder verkittet ist und einen von einem Befestigungsflansch (23) begrenzten Aufnahmekonus (22) hat, in den ein mit einem Kunststoffschlauch (30) verbundener Abgabestutzen (33) passend einsteckbar ist, und daß der Abgabestutzen von einer Schraubkappe (34) umgeben ist, die unter Einziehen des Abgabestutzens (33) in den Aufnahmekonus (22) auf den Befestigungsflansch (23) des Sockelteils (2) aufschraubbar ist.

5. Injektionsvorrichtung nach Anspruch 4, dadurch gekennzeichnet, daß der Kunststoffschlauch (30) an dem dem Abgabestutzen (33) entgegengesetzten Ende ein Kupplungsstück (32) mit einem von einem Befestigungsflansch begrenzten Aufnahmekonus aufweist, der nach Form und Ausbildung dem Sockelteil (2) entspricht, und daß der Kunststoffschlauch zusammen mit den ihm zugeordneten Anschlußteilen (31, 32) eine Verlängerung (3) von 25 bis 35 cm Länge zum Anschluß des Spritzenabgabestutzens an den Kanülensockel (2) bildet.

**Claims**

1. Injection assembly for blocking peripheral nerves, e.g. for plexus anaesthesia, comprising an injection needle (1) of metal provided on one end with a needle tip (10) and on the opposite end with a base portion (2) adapted to be connected with a syringe, characterized in that the needle tip (10) is defined by a ground surface (12) inclined at an angle (δ) ranging between 40 to 60° with respect to the cannula axis (13).

2. Injection assembly according to claim 1, characterized in that the angle (δ) of the needle tip (10) ranges between 45 and 50°.

3. Injection assembly according to claim 1 or 2, characterized in that the outermost end of the needle tip (10) is slightly rounded and dulled respectively.

4. Injection assembly according to one of claims 1 to 3 characterized in that a base portion (2) of plastic is glued or cemented to the end of the needle opposite to said needle tip (10) and has a tapered socket (22) terminating in a mounting flange (23), said tapered socket being adapted to receive a tapered chuck (33) coupled to a flexible plastic tube (30), and in that said chuck is surrounded by a screw cap (34) which is adapted to be screwed to said mounting flange (23) of said base portion (2), thereby drawing the tapered chuck (33) into the tapered socket (22).

5. Injection assembly according to claim 4, characterized in that said plastic tube (30) comprises at its end opposite to said tapered chuck (33) a fitting (32) said fitting having a tapered socket limited outwardly by a fastening flange said fastening flange having the same shape and construction as said base portion (2) and in that said plastic tube along with said fittings (31, 32) associated thereto form an extension (3) which has a length ranging from 20 to 35 cm for coupling the chuck of the syringe to the tapered socket (2) of said cannula.

## Revendications

1. Dispositif d'injection pour le blocage des nerfs périphériques, par exemple pour anesthésie plexulaire, avec une aiguille d'injection (1) en métal, qui est munie, à une extremité, d'une pointe d'aiguille (10) et, à l'extrémité opposée, d'un culot (2) destine au raccordement d'une seringue, caractérisé par le fait que la pointe d'aiguille (10) est formée par une section polie (12) inclinée sous un angle (δ) de 40 à 60° par rapport à l'axe de canule (13).

2. Dispositif d'injection selon la revendication 1, caractérisé par le fait que l'angle (δ) de la pointe d'aiguille (10) est comprise entre 45 et 50°.

3. Dispositif d'injection selon la revendication 1 ou 2, caractérisé par le fait que l'extremité la plus extérieure de la pointe d'aiguille (10) est legèrement arrondie ou emoussée.

4. Dispositif d'injection selon l'une des revendications 1 à 3, caractérisé par le fait qu'un socle en matière plastique (2) est collé ou mastique sur l'extremite d'aiguille opposée à la pointe d'aiguille (10) et possède un cône de reception (22) limite par une bride de fixation (23), cône de reception (22) dans lequel vient s'enficher convenablement un raccord de distribution (33) raccorde à un flexible en matière plastique (30), et que le raccord de distribution est entoure d'un bouchon filete (34) qui vient se visser sur la bride de fixation (23) du culot (2) lors de l'insertion du raccord de distribution (33) dans le cône de reception (22).

5. Dispositif d'injection selon la revendication 4, caracterise par le fait que le flexible en matière plastique (30) presente, à l'extremite opposée au raccord de distribution (33), une pièce d'accouplement (32) munie d'un cône de reception limite par une bride de fixation, cône de reception dont la forme et la configuration correspondent au culot (2), et que le flexible en matière plastique constitue, avec les pièces de raccordement (31,32) qui lui sont subordonnées, une rallonge (3) de 25 à 35 cm de long pour le raccordement du raccord distributeur de la serirgue sur le culot de canule (2).

0 059 784

Fig. 2

Fig.1